(19) Europäisches Patentamt
European Patent Office
Office européen des brevets

(11) EP 4 570 296 A1

(12) EUROPEAN PATENT APPLICATION
published in accordance with Art. 153(4) EPC

(43) Date of publication:
18.06.2025 Bulletin 2025/25

(51) International Patent Classification (IPC):
A61M 25/10 (2013.01)

(21) Application number: 23871547.8

(52) Cooperative Patent Classification (CPC):
A61M 25/10

(22) Date of filing: 17.08.2023

(86) International application number:
PCT/JP2023/029666

(87) International publication number:
WO 2024/070305 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.09.2022 JP 2022155931

(71) Applicant: Terumo Kabushiki Kaisha
Tokyo 151-0072 (JP)

(72) Inventors:
• FUKUDA, Keiji
Fujinomiya-shi, Shizuoka 418-0015 (JP)
• ONO, Kensuke
Fujinomiya-shi, Shizuoka 418-0015 (JP)

(74) Representative: Casalonga
Casalonga & Partners
Bayerstraße 71/73
80335 München (DE)

(54) BALLOON CATHETER

(57) To provide a balloon catheter having excellent pressure resistance and operability by being thinned while suppressing a decrease in pressure resistance strength accompanying an increase in diameter of the balloon.

A balloon 140 of a balloon catheter 100 includes at least an inner layer 141 and an outer layer 143, has an area draw-down ratio (ADDR) of 2.91 or more and 2.97 or less, the area draw-down ratio (ADDR) being determined by a ratio of the cross-sectional area of a tubular parison 300 disposed in a mold 200 and blow-molded in a balloon shape to a cross-sectional area of the balloon 140 after blow molding, and has a layer provided outside the inner layer 141 and having an elongation rate at a breaking point lower than the elongation rate at a breaking point of the inner layer 141.

FIG. 3

**Description**

Technical Field

The present invention relates to a balloon catheter.

Background Art

A balloon catheter is widely known as a device used for a surgery of expanding a lesion (such as a stenosis) formed in a body lumen such as a blood vessel or placement of a stent or the like (for example, Patent Literature 1). The balloon catheter can be inserted into a blood vessel via a guiding catheter in a state where the balloon is contracted to reach a lesion, and the balloon can be expanded to push and extend the lesion (for example, Patent Literature 1).

Citation List

Patent Literature

Patent Literature 1: WO 2019/234784 A

Summary of Invention

Technical Problem

Since a balloon catheter is expanded/contracted within a predetermined range on the basis of the properties of the lesion and the blood vessel, when the expansion diameter of the balloon is expanded, the expansion/contraction range of the balloon (the expandable/contractible range of the balloon) can be widened, and the degree of freedom of the surgery can be increased.

For example, in a case where the diameter of a balloon catheter for treatment of PTCA (the outer diameter of the balloon at the time of expansion: 4.0 mm) is increased to 6.0 mm, the film thickness at the time of balloon molding becomes too thin as the diameter is increased, and the pressure resistance strength may decrease. In order to maintain the pressure resistance strength, it is conceivable to increase the thickness (wall thickness) of the original tube (tubular parison) before molding, but if the wall thickness of the tubular parison is increased, the film thickness itself of the balloon, which is a molded article, becomes thick and hard. When the film thickness of the balloon becomes thick and hard, the resistance of the reduced diameter portion of the balloon increases when the balloon that has been contracted (deflated) is drawn into a guiding catheter or pulled back inside the placed stent, which leads to a delay in a surgery.

At least one embodiment of the present invention has been made in view of the above circumstances, and specifically, it is an object of the present invention to provide a balloon catheter capable of securing flexibility due to thinning while suppressing a decrease in pressure resistance strength when a diameter of a balloon is increased, and achieving both pressure resistance and good operability.

Solution to Problem

In view of the above problems, the inventors of the present application have focused on a layer structure of a balloon in order to achieve both pressure resistance and operability (in particular, draw-in operability to a guiding catheter or the like) of the balloon, and have found the following events from a comparison between a two-layer structure balloon and a three-layer structure balloon.

It has been found that, in a balloon having a two-layer structure having an inner layer (for example, nylon elastomer) and an outer layer (for example, nylon), when a ratio between an orientation in the circumferential direction of the balloon (hereinafter, referred to as "circumferential orientation") and an orientation in the axial direction of the balloon (hereinafter, referred to as "axial orientation") is compared, the circumferential orientation is higher than the axial orientation, and there is a high risk that the balloon inflates in the circumferential direction before the balloon fully extends in the axial direction and a lateral crack (circumferential burst) occurs.

It has been found that a balloon having a three-layer structure of an inner layer (for example, nylon elastomer), an intermediate layer (for example, nylon), and an outer layer (for example, nylon elastomer) can enhance axial orientation by disposing the outer layer, and can eliminate the risk of lateral cracking by being fully extended in the axial direction before being inflated in the circumferential direction. However, the outer layer itself does not contribute much to improvement in pressure resistance strength, and it is necessary to increase the thickness of the outer layer in order to increase the pressure resistance strength of the balloon.

As a result of intensive studies on balloon characteristics necessary for improving pressure resistance and operability associated with an increase in diameter of a balloon based on the obtained findings, the inventors of the present invention have found that in a balloon having a multilayer structure including at least an inner layer and an outer layer, when dimensions of the balloon are optimized, an area draw-down ratio ((ADDR): the ratio of the cross-sectional area of a tubular parison before blow molding to the cross-sectional area of the balloon after blow molding) falls within a specific range, and an elongation rate at a breaking point satisfies a specific condition, pressure resistance strength capable of suppressing the risk of lateral cracking can be maintained while flexibility due to thinning is secured, and the present invention has been completed.

The present invention is (1) a balloon catheter including a balloon having a main body with an expandable and contractible multilayer film structure, in which the balloon includes at least an inner layer and an outer layer, has an area draw-down ratio (ADDR) of more than 2.80 and less than 3.00, the area draw-down ratio (ADDR) being determined by a ratio of a cross-sectional area of a tubular parison disposed in a mold and blow-molded in a balloon shape to a cross-sectional area of the balloon after blow molding, and includes a layer provided outside the inner layer and having an elongation rate at a breaking point lower than an elongation rate at a breaking point of the inner layer.

Here, embodiments of the present invention can be configured as follows.

(2) In the balloon catheter according to (1), the area draw-down ratio is preferably 2.91 or more and 2.97 or less.

(3) In the balloon catheter according to (1) or (2), a film thickness of the main body of the balloon after blow molding is preferably 26.6 $\mu$m or more and 36.0 $\mu$m or less.

(4) In the balloon catheter according to (1) or (2), a film thickness of the main body of the balloon after blow molding is preferably 32.3 $\mu$m or more and 33.0 $\mu$m or less.

(5) In the balloon catheter according to any one of (1) to (4), the balloon preferably has a lateral cracking risk value of 1.40 or less, the lateral cracking risk value being obtained from a ratio of a circumferential orientation ratio to an axial orientation ratio.

(6) In the balloon catheter according to any one of (1) to (5), the main body of the balloon preferably has a three-layer structure in which the inner layer, an intermediate layer, and the outer layer are stacked in this order.

(7) In the balloon catheter according to (6), an elongation rate at a breaking point of the intermediate layer is preferably lower than each of the elongation rate at the breaking point of the inner layer and an elongation rate at a breaking point of the outer layer.

(8) In the balloon catheter according to (6) or (7), an elongation rate at a breaking point of the outer layer is preferably equal to or more than the elongation rate at the breaking point of the inner layer.

(9) In the balloon catheter according to (8), the elongation rate at the breaking point of the inner layer is preferably equal to the elongation rate at the breaking point of the outer layer.

(10) In the balloon catheter according to any one of (6) to (9), in the balloon, cross-sectional area percentages of the inner layer, the intermediate layer, and the outer layer in the tubular parison are preferably 22.5%, 53.5%, and 24.0%, respectively.

(11) In the balloon catheter according to any one of (6) to (10), it is preferable that in the main body of the balloon, the inner layer includes a nylon elastomer, the intermediate layer includes a nylon, and the outer layer includes a nylon elastomer.

Advantageous Effects of Invention

According to at least one embodiment of the present invention, it is possible to provide a balloon catheter capable of securing flexibility due to thinning while suppressing a decrease in pressure resistance strength when a diameter of a balloon is increased, and achieving both pressure resistance and good operability.

Brief Description of Drawings

Fig. 1 is a schematic configuration diagram of a balloon catheter according to the present embodiment.
Fig. 2 is a cross-sectional view of the periphery of a distal portion of the balloon catheter according to the present embodiment.
Fig. 3 is a cross-sectional view of a tubular parison before blow molding of a balloon according to the present embodiment.
Fig. 4 is a schematic cross-sectional view of a state in which the tubular parison is disposed in a mold.
Fig. 5 is a graph illustrating a relationship between an area draw-down ratio and a film thickness.

Description of Embodiments

Hereinafter, a mode for carrying out the present invention will be described in detail with reference to the drawings. Embodiments herein described are illustrated to embody the technical idea of the present invention and do not limit the present invention. Other feasible embodiments, examples, operation technologies and the like that could be conceived by those skilled in the art without departing from the gist of the present invention are all included in the scope and gist of the present invention and included in the invention recited in claims and the scope of equivalents thereof.

Moreover, for convenience of illustration and ease of understanding, the drawings attached to the present specification might be schematically represented by changing a scale, an aspect ratio, a shape and the like from actual ones as appropriate, but are merely examples, and do not limit the interpretation of the present invention.

Note that, in the following description, ordinal numerals such as "first" and "second" will be given, but are used for convenience and do not define any order unless otherwise specified.

[Configuration]

First, a configuration of a balloon catheter 100 according to the present embodiment will be described.

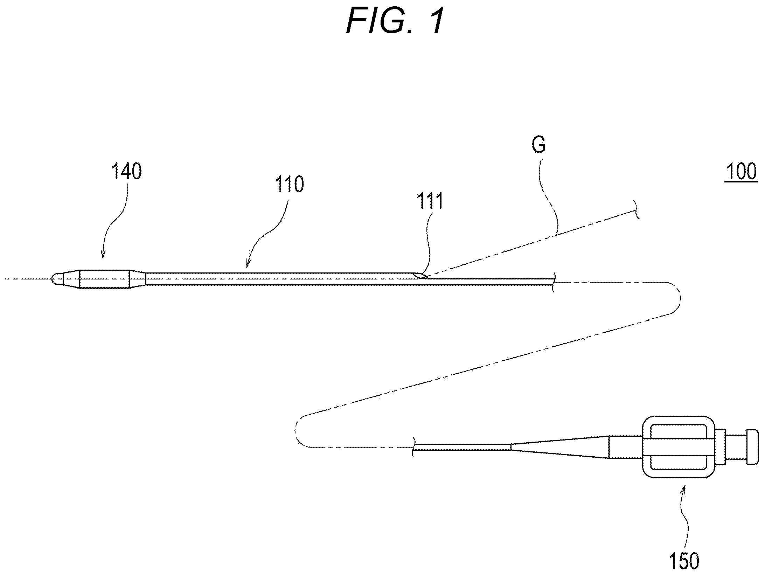

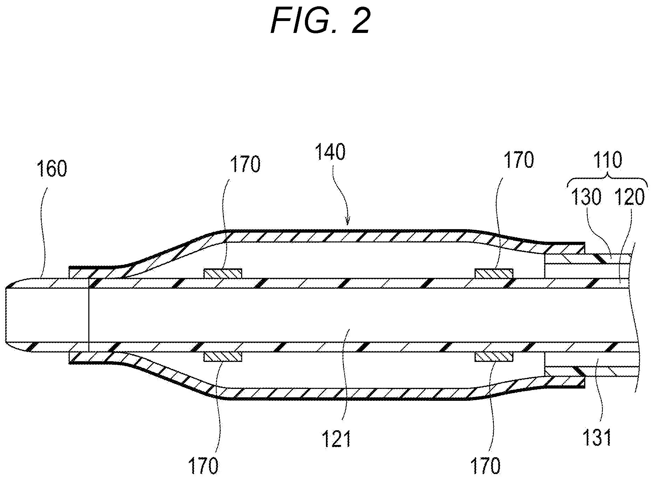

As illustrated in Fig. 1 or 2, the balloon catheter 100 is a medical device that treats a lesion such as a stenosis formed in a body lumen by expanding a balloon 140 disposed on a distal side of a shaft 110 to push and extend the lesion.

The balloon catheter 100 can be configured as, for example, a balloon catheter for PTCA treatment used to extend a lesion of a coronary artery. However, the balloon catheter 100 can be configured to be used for the purpose of treating and improving a lesion formed in a biological organ such as another blood vessel, a bile duct, a trachea, an esophagus, another digestive tract, a urethra, an ear and nose lumen, or another organ.

In the following description, a side on which the balloon 140 is disposed is referred to as a "distal side" of the balloon catheter 100, a side on which a hub 150 is disposed is referred to as a "proximal side" of the balloon catheter 100, and a direction in which the shaft 110 extends is referred to as an "axial direction". Furthermore, unless otherwise specified, a "distal portion" represents a certain range including a distal end (the most distal end) and its periphery, and a "proximal portion" represents a certain range including a proximal end (the most proximal end) and its periphery.

The balloon catheter 100 is configured as a so-called "rapid exchange type catheter device" in which a guidewire port 111 from which a guidewire G is led out is provided closer to the distal portion side of the shaft 110. Note that the balloon catheter 100 can also be configured as what is called "an over-the-wire type catheter device" having a guidewire lumen 121 extending from the distal end to the proximal end of the shaft 110.

The balloon catheter 100 may be provided with the hub 150 at the proximal portion of the shaft 110, as illustrated in Fig 1. The hub 150 is configured to be connectable to a connector (Y connector) known in the medical field, and can be connected to a supply device (not illustrated) such as an indeflator for supplying a pressurizing medium via the connector in a liquid-tight and air-tight manner.

As illustrated in Fig. 2, the shaft 110 includes an inner tube 120 in which the guidewire lumen 121 through which the guidewire G is inserted is formed, and an outer tube 130 forming a pressurizing medium lumen 131 between the outer tube 130 and the inner tube 120. Through the pressurizing medium lumen 131, a pressurizing medium can flow. The shaft 110 has a double tube structure in which the inner tube 120 is inserted into the outer tube 130 such that the inner tube 120 and the outer tube 130 are arranged in a concentric manner.

The balloon 140 is joined to the distal portion of the inner tube 120 in a liquid-tight and air-tight manner by a known method such as welding. The balloon 140 has a distal portion joined to the inner tube 120 and a proximal portion joined to the outer tube 130.

A distal tip 160 can be attached to the distal end of the inner tube 120 to protect a biological organ (for example, an inner wall of a blood vessel) from damage when, for example, the distal end of the balloon catheter 100 comes into contact with the biological organ. The distal tip 160 can include, for example, a more flexible material than the inner tube 120.

The inner tube 120 can be provided with a contrast marker portion 170. For example, the contrast marker portions 170 can be disposed at a position indicating a boundary with the distal side of the balloon 140 in the inner tube 120 and at a position indicating a boundary with the proximal side of the balloon 140 in the inner tube 120.

Examples of the material used in the inner tube 120 and the outer tube 130 include polyolefins such as polyethylene, polypropylene, ethylene-propylene copolymer, and ethylene-vinyl acetate copolymer, thermoplastic resins such as soft polyvinyl chloride, various rubbers such as silicone rubber and latex rubber, various elastomers such as polyurethane elastomers, polyamide elastomers, and polyester elastomers, and crystalline plastics such as polyamide, crystalline polyethylene, and crystalline polypropylene. In these materials, for example, antithrombotic substance such as heparin, prostaglandin, urokinase, or arginine derivatives can be blended to obtain a material having antithrombogenicity.

The balloon 140 is disposed on the distal side of the shaft 110 (the distal side of the inner tube 120), and has a space portion which is formed between the balloon 140 and the inner tube 120 and into which the pressurizing medium can flow. The balloon 140 expands when the pressurizing medium flows into the space portion. When the balloon 140 is expanded, the balloon catheter 100 expands a lesion formed in the body lumen by pushing a part of the balloon 140 against the lesion to push and extend the lesion.

As a material constituting the balloon 140, for example, an organic polymer material can be used. Specifically, it is possible to use an elastic resin such as a polymer material such as polyolefins (for example, polyethylene, polypropylene, polybutene, ethylene-propylene copolymer, ethylene-vinyl acetate copolymer, ionomer, or a mixture of two or more types thereof), polyvinyl chloride, polyamides, (for example, nylon such as nylon 6, nylon 6,6, nylon 6,10, nylon 12), polyamide elastomer, nylon elastomer, polyurethane, polyurethane elastomer, polyimide, or fluororesin, or a mixture thereof, or two or more types of the polymer materials described above.

The balloon 140 has a multilayer film structure including at least an inner layer 141 and an outer layer 143.

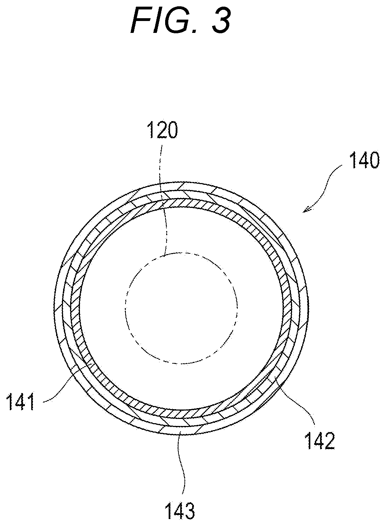

The balloon 140 can have a three-layer structure including the inner layer 141, an intermediate layer 142, and the outer layer 143 as illustrated in Fig. 3. In the balloon 140 illustrated in Fig. 3, the inner layer 141 can include a nylon elastomer, the intermediate layer 142 can include nylon, and the outer layer 143 can include a nylon elastomer. Since the balloon 140 has a three-layer structure, by disposing the outer layer 143, it is possible to increase axial orientation and reduce the risk of lateral cracking (the risk of occurrence of a circumferential burst) by fully extending in the axial direction before the balloon 140 inflates in the circumferential direction. Note that the material configuration of each layer of the balloon 140 is not limited to the above configuration.

The pressurizing medium (for example, fluid such as saline or contrast) used for expansion of the balloon 140 can flow into the pressurizing medium lumen 131 of the shaft 110 through internal space (lumen) of the hub 150. The pressurizing medium is supplied to the space portion of the balloon 140 via the pressurizing medium lumen 131.

A coating that covers the outer surface of the balloon 140 can be formed. The coating may include, for example, a hydrophilic coating layer that improves slidability of the balloon 140 or a drug coating layer containing a predetermined drug. A specific material for forming the hydrophilic coating layer or the drug coating layer is not particularly limited.

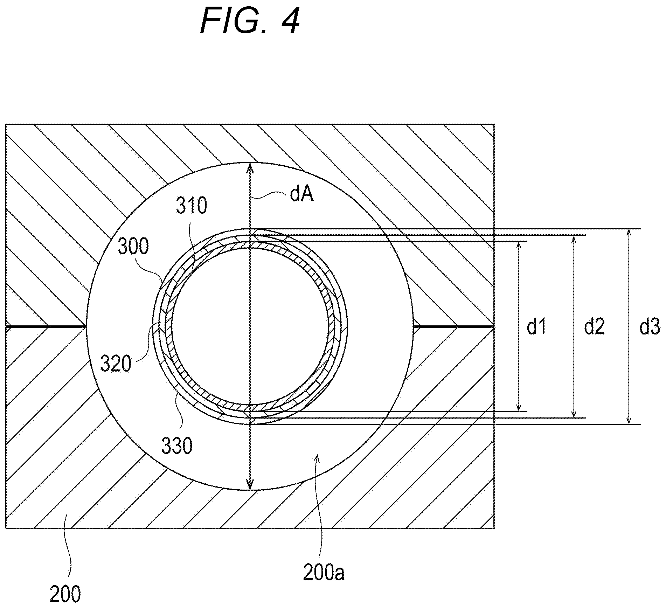

The balloon 140 is preliminarily molded by manufacturing a tubular parison 300 (original tube) including the elastic resin described above by injection molding, and subjecting the tubular parison 300 to known stretch blow molding (for example, biaxial stretch blow molding). An inner layer 310 of the tubular parison 300 illustrated in Fig. 4 becomes the inner layer 141 of the balloon 140 after blow molding, an intermediate layer 320 becomes the intermediate layer 142 of the balloon 140 after blow molding, and an outer layer 330 becomes the outer layer 143 of the balloon 140 after blow molding. Thereafter, the balloon 140 that has been preliminarily molded can be subjected to a shaping step using a mold to form a plurality of blade portions that protrudes radially from the inner tube 120 and is folded so as to be wound around the outer periphery during contraction. As a result, since the blade portions are folded and reduced in diameter upon contraction after expansion, the passability with respect to a blood vessel and the guiding catheter can be enhanced.

In the balloon 140 according to the present embodiment, in order to realize pressure resistance and operability by thinning the balloon 140 while suppressing a decrease in pressure resistance strength due to an increase in the diameter of the balloon outer diameter, and in particular, smooth surgery by reducing resistance at the time of drawing the balloon 140 into the guiding catheter after contraction and diameter reduction, the dimensions of the layer structure are optimized by defining the following parameters. Hereinafter, the layer structure of the balloon 140 will be described in detail.

The balloon 140 has an outer diameter at the time of expansion (outer diameter when expanded at recommended expansion pressure (NP), hereinafter also referred to as "outer diameter at the time of expansion at NP") of more than 4.0 mm and 6.0 mm or less, preferably 5.0 mm or more and 6.0 mm or less, more preferably 5.5 mm or more and 6.0 mm or less, and most preferably 6.0 mm.

The balloon 140 has an area draw-down ratio (ADDR) of more than 2.80 and less than 3.00, preferably 2.91 or more and 2.97 or less. In the present specification, the "area draw-down ratio (ADDR)" is a value determined by a ratio of the cross-sectional area of the tubular parison 300 to the cross-sectional area of the balloon 140 obtained by blow molding the tubular parison 300 disposed in a mold 200.

Here, when the area draw-down ratio is less than 2.80, even if the outer layer 143 is disposed, the axial orientation does not increase, and the balloon 140 inflates in the circumferential direction before fully extending in the axial direction, so that the risk of lateral cracking cannot be suppressed. When the area draw-down ratio is 3.00 or more, the balloon 140 does not inflate during blow molding, resulting in molding failure. In addition, when the film thickness of the balloon 140 is less than 26.6 $\mu$m, the pressure resistance is lowered and the risk of bursting is increased, and when the film thickness exceeds 36.0 $\mu$m, the outer diameter of the reduced diameter portion of the contracted balloon 140 is increased and the draw-in resistance is increased, so that the operability is deteriorated.

The balloon 140 has a film thickness of 26.6 $\mu$m or more and 36.0 $\mu$m or less, preferably 32.3 $\mu$m or more and 33.0 $\mu$m or less after the tubular parison 300 is set in the mold 200 and blow-molded.

The balloon 140 includes, at least outside the inner layer 141, a layer having an elongation rate at a breaking point lower than that of the inner layer 141. In the balloon 140, the elongation rate at a breaking point is higher in the outer layer 143 than in the intermediate layer 142 (intermediate layer<outer layer), and is higher in the inner layer 141 than in the intermediate layer 142 (inner layer>intermediate layer). That is, the elongation rate at the breaking point of the intermediate layer 142 is lower than each of those of the inner layer 141 and the outer layer 143. In the balloon 140, the elongation rates at the breaking points of the inner layer 141 and the outer layer 143 may be equal, or the elongation rate at the breaking point in the outer layer 143 may be higher. Note that since the balloon 140 only needs to include a layer having a lower elongation rate at a breaking point than that of the inner layer 141 at least on the outside of the inner layer 141, for example, a layer having an elongation rate at a breaking point lower than that of the inner layer 141 can be included between the inner layer 141 and the intermediate layer 142, between the intermediate layer 142 and the outer layer 143, or further on the outer side of the outer layer 143.

From the viewpoint of pressure resistance and operability, the balloon 140 has a lateral cracking risk value of 1.40 or less, the lateral cracking risk value being obtained from the ratio of the circumferential orientation ratio to the axial orientation ratio. The lateral cracking risk value is a numerical value of the risk of occurrence of a lateral crack at the time of expansion, and can be acquired by the following method. Note that an example of the method of measuring the lateral cracking risk value will be described in Examples.

<Procedure 1>

Using the following Formula (1), the average balloon film intrinsic refractive index is calculated from the cross-sectional area percentage of each layer of the tubular parison 300 having a three-layer structure. In Formula (1), "a" represents the refractive index of the outer layer, "a1" represents the cross-sectional area percentage of the outer layer, "b" represents the refractive index of the intermediate layer, "b1" represents the cross-sectional area ratio of the intermediate layer, "c" represents the refractive index of the inner layer, and "c1" represents the cross-sectional area percentage of the inner layer.

[Mathematical Formula 1]

$$n = \frac{a \times a1 + b \times b1 + c \times c1}{3} \qquad \cdots (1)$$

<Procedure 2>

The balloon 140 after blow molding is cut and spread out to form a rectangular test piece, and the test piece is attached onto a slide glass with a tape adhesive to prepare a measurement sample. A phase difference distribution image of the prepared test piece is acquired using a two-dimensional birefringence evaluation system (for example, WPA-200: manufactured by Photonics Lattice, Inc.), and two points in each of three areas, that is, a balloon distal portion, a balloon central portion, and a balloon proximal portion in the test piece are analyzed to extract a phase difference average value (retardation) of each area.

<Procedure 3>

Birefringence Δn is calculated from the ratio (retardation average value/film thickness) of the average value (any of the balloon distal portion, the balloon central portion, and the balloon proximal portion) of the retardation obtained in Procedure 2 to the film thickness of the balloon 140 after blow molding measured by a thickness measuring instrument (for example, ID-F125 thickness gauge: manufactured by Mitutoyo Corporation).

<Procedure 4>

A circumferential refractive index nr and an axial refractive index nl are calculated from the birefringence Δn obtained in Procedure 3 using the following Formulas (2) and (3). In Formulas (2) and (3), "nd" is the refractive index in the thickness direction, and an assumed value (1.501) empirically determined for nylon-based material can be substituted.

[Mathematical Formula 2]

$$nr = \frac{2\Delta n \pm \sqrt{24n^2 - 4\Delta n^2 - 8nd^2}}{4} \quad \cdots (2)$$

$$nl = \frac{-2\Delta n \pm \sqrt{24n^2 - 4\Delta n^2 - 8nd^2}}{4} \quad \cdots (3)$$

<Procedure 5>

Using the following Formulas (4) to (6), the orientation ratio r% in the circumferential direction and the orientation ratio l% in the axial direction are calculated from the refractive indices in the respective directions obtained in Procedure 4. In Formulas (4) to (6), since the intrinsic birefringence of nylon 6 is 0.072 and the intrinsic birefringence of polyethylene is 0.66, 0.07 can be substituted as an approximate value into "n*". "d%" represents the orientation ratio in the thickness direction. "|nl - nd|" represents birefringence $\Delta$n.

[Mathematical Formula 3]

$$d\% = \frac{1 - \frac{|nl - nd|}{n^*} - \frac{|nr - nd|}{n^*}}{3} \quad \cdots (4)$$

$$l\% = d\% + \frac{|nl - nd|}{n^*} \quad \cdots (5)$$

$$r\% = d\% + \frac{|nr - nd|}{n^*} \quad \cdots (6)$$

<Procedure 6>

The ratio (r%/l%) of the orientation ratio r% in the circumferential direction to the orientation ratio l% in the axial direction obtained in Procedure 5 is determined, and the obtained value is defined as a lateral cracking risk value. The above 3 to 6 are calculated for each of the balloon distal portion, the balloon central portion, and the balloon proximal portion to obtain an average value of the lateral cracking risk values of the test piece, and the average value can be acquired as the lateral cracking risk value of the test piece (balloon 140).

In the balloon 140, a lateral crack is likely to occur if the lateral cracking risk value exceeds 1.40. Therefore, the balloon 140 is not suitable as a product from the viewpoint of pressure resistance and operability. Therefore, the balloon 140 preferably has a lateral cracking risk value of 1.40 or less.

The balloon 140 can reduce the risk of lateral cracking by adjusting the cross-sectional area percentage of each layer. In the balloon 140, if the cross-sectional area percentages are adjusted by decreasing the amount of the outer layer 143 by a predetermined amount and increasing the amount of the inner layer 141 by a predetermined amount in a state where the amount of the intermediate layer 142 is fixed, the expansion timing at the time of expansion can be delayed, so that expansion in the axial direction is promoted and the risk of lateral cracking can be reduced. The balloon 140 can have cross-sectional area percentages of the inner layer 141, the intermediate layer 142, and the outer layer 143 of 22.5%, 53.5%, and 24.0%, respectively, in the state of the tubular parison 300.

[Operational Effects]

As described above, the balloon catheter 100 according to the present embodiment includes the balloon 140 having the main body with an expandable and contractible multilayer film structure, in which the balloon 140 includes at least the inner layer 141 and the outer layer 143, has an area draw-down ratio (ADDR) of more than 2.80 and less than 3.00, the area draw-down ratio (ADDR) being determined by the ratio of the cross-sectional area of the tubular parison 300 disposed in the mold 200 and blow-molded in a balloon shape to the cross-sectional area of the balloon 140 after blow molding, and has a layer provided outside the inner layer 141 and having an elongation rate at a breaking point lower than that of the inner layer 141.

Furthermore, in the balloon catheter 100 according to the present embodiment, the area draw-down ratio of the balloon 140 is preferably 2.91 or more and 2.97 or less, and the film thickness of the main body of the balloon 140 after blow molding can be 26.6 $\mu$m or more and 36.0 $\mu$m or less, and preferably 32.3 $\mu$m or more and 33.0 $\mu$m or less.

As a result, even when the diameter of the balloon 140 is increased, the risk of lateral cracking is suppressed while maintaining the pressure resistance strength, and further, flexibility can be secured by thinning. Therefore, it is possible to provide the balloon catheter 100 having both pressure resistance and good operability.

In addition, in the balloon catheter 100 according to the present embodiment, the balloon 140 can have a lateral cracking risk value of 1.40 or less, the lateral cracking risk value being obtained from the ratio of the circumferential orientation ratio to the axial orientation ratio.

As a result, it is possible to provide the balloon catheter 100 including the balloon 140 in which the risk of lateral cracking at the time of expansion is reduced.

Furthermore, in the balloon catheter 100 according to the present embodiment, the main body of the balloon 140 can have a three-layer structure in which the inner layer 141, the intermediate layer 142, and the outer layer 143 are stacked in this order. In addition, it is possible to adopt a configuration in which the elongation rate at the breaking point of the intermediate layer 142 is lower than each of those of the inner layer 141 and the outer layer 143, a configuration in which the elongation rate at the breaking point of the outer layer 143 is equal to or higher than that of the inner layer 141, or a configuration in which the elongation rate at the breaking point of the inner layer 141 is equal to that of the outer layer 143.

Since the balloon 140 has a three-layer structure, the balloon 140 can be fully extended in the axial direction before being inflated in the circumferential direction when expanded, so that the risk of lateral cracking can be reduced.

Furthermore, in the balloon catheter 100 according to the present embodiment, the balloon 140 can have the cross-sectional area percentages of the inner layer 141, the intermediate layer 142, and the outer layer 143 in the tubular parison 300 of 22.5%, 53.5%, and 24.0%, respectively.

As a result, the amount of the inner layer 141 can be increased and the amount of the outer layer 143 can be decreased in a state where the amount of the intermediate layer 142 is fixed, and the expansion timing at the time of expansion can be delayed, so that expansion in the axial direction is promoted and the risk of lateral cracking can be reduced.

Furthermore, in the balloon catheter 100 according to the present embodiment, in the main body of the balloon 140, the inner layer 141 can include a nylon elastomer, the intermediate layer 142 can include a nylon, and the outer layer 143 can include a nylon elastomer.

Accordingly, since the balloon 140 can be fully extended in the axial direction before being inflated in the circumferential direction, it is possible to provide the balloon catheter 100 having excellent pressure resistance and operability.

Examples

Hereinafter, while the present invention will be specifically described with reference to Examples, the scope of the present invention is not limited to the following Examples.

[Test 1. Measurement test of area draw-down ratio and film thickness]

A measurement test of the area draw-down ratio (ADDR) and the film thickness was performed as follows.

<1-1. Production of sample>

<Example A1>

In Example A1, a tubular parison having a three-layer structure in which an inner layer (inner diameter: 0.82 mm) included a nylon elastomer (Product name: Grilflex ELG6260, manufactured by Ems-chemie Holding AG), an intermediate layer (inner diameter: 1.09 mm, outer diameter: 1.56 mm) included a nylon (Product name: Grilamid L25, manufactured by Ems-chemie Holding AG), and an outer layer (outer diameter: 1.73 mm) included a nylon elastomer (Product name: Grilflex ELG6260, manufactured by Ems-chemie Holding AG) was produced by extrusion molding, and this tubular parison was disposed in a mold and blow-molded (mold temperature: 115°C, heating time: 30 seconds, pressure: 3.0 MPa) to obtain a sample having an outer diameter at the time of expansion at NP of 6.0 mm.

<Example A2>

In Example A2, a tubular parison having a three-layer structure in which an inner layer (inner diameter: 0.75 mm) included a nylon elastomer (Product name: Grilflex ELG6260, manufactured by Ems-chemie Holding AG), an intermediate layer (inner diameter: 1.00 mm, outer diameter: 1.43 mm) included a nylon (Product name: Grilamid L25, manufactured by Ems-chemie Holding AG), and an outer layer (outer diameter: 1.58 mm) included a nylon elastomer (Product name: Grilflex ELG6260, manufactured by Ems-chemie Holding AG) was produced by extrusion molding, and this tubular parison was disposed in a mold and blow-molded (mold temperature: 115°C, heating time: 30 seconds, pressure: 3.0 MPa) to obtain a sample having an outer diameter at the time of expansion at NP of 5.5 mm.

<Example A3>

In Example A3, a tubular parison having a three-layer structure in which an inner layer (inner diameter: 0.68 mm) included a nylon elastomer (Product name: Grilflex ELG6260, manufactured by Ems-chemie Holding AG), an intermediate layer (inner diameter: 0.91 mm, outer diameter: 1.30 mm) included a nylon (Product name: Grilamid L25, manufactured by Ems-chemie Holding AG), and an outer layer (outer diameter: 1.44 mm) included a nylon elastomer (Product name: Grilflex ELG6260, manufactured by Ems-chemie Holding AG) was produced by extrusion molding, and this tubular parison was disposed in a mold and blow-molded (mold temperature: 115°C, heating time: 30 seconds, pressure: 3.0 MPa) to obtain a sample having an outer diameter at the time of expansion at NP of 5.0 mm.

The elongation rates at the breaking points in each of Examples 1 to 4 were in the relationship of the inner layer > the intermediate layer, the intermediate layer < the outer layer, and the inner layer = the outer layer.

<1-2. Measurement method>

The area draw-down ratio was determined from the ratio of the cross-sectional area of the tubular parison of each sample to the cross-sectional area of the balloon after blow molding. For the film thickness, the film thickness of each sample after blow molding was measured with a thickness measuring instrument (Product name: Digimatic indicator ID-H, manufactured by Mitutoyo Corporation), and a value for one sample was calculated from the thickness of the film thickness of two samples.

Measurement results are presented in Table 1 below. Table 1 indicates the upper and lower limit values of the area draw-down ratio and the upper and lower limit values of the film thickness ($\mu$m) of each of the produced samples.

[Table 1]

|  | Example A1 | Example A2 | Example A3 |
|---|---|---|---|
| Outer diameter (mm) | 6.0 | 5.5 | 5.0 |
| ADDR upper limit value | 2.97 | 2.94 | 2.99 |
| ADDR lower limit value | 2.90 | 2.91 | 2.81 |
| Film thickness upper limit value | 33.0 | 29.8 | 28.5 |
| Film thickness lower limit value | 32.3 | 29.6 | 26.6 |

<1-3. Results>

As indicated in Table 1, in each of Example A1, Example A2, and Example A3, the area draw-down ratio was more than 2.80 and less than 3.00, and the film thickness was 26.6 $\mu$m or more and 36.0 $\mu$m or less.

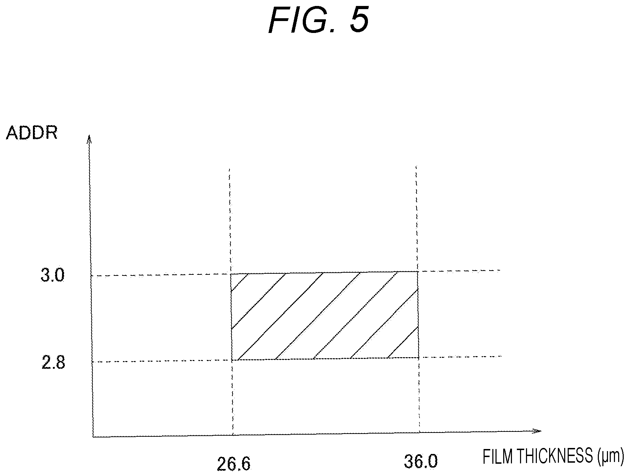

Fig. 5 is a graph illustrating a relationship between the area draw-down ratio of the balloon 140 and the film thickness. The hatched region illustrated in the graph of Fig. 5 is a region partitioned such that the area draw-down ratio is more than 2.80 and less than 3.00 and the film thickness is 26.6 $\mu$m or more and 36.0 $\mu$m or less, and products falling within this range can be evaluated as products excellent in pressure resistance and operability. In each of Example A1, Example A2, and Example A3, the area draw-down ratio was more than 2.80 and less than 3.00, and the film thickness was 26.6 $\mu$m or more and 36.0 $\mu$m or less, and each of Example A1, Example A2, and Example A3 was within the hatched region in Fig. 5. Therefore, it was confirmed that Example A1, Example A2, and Example A3 were balloons having both pressure resistance and good operability in which the risk of lateral cracking was suppressed while maintaining the pressure resistance strength even when the diameter of the balloon was increased, and further, flexibility could be secured by thinning.

[Test 2. Lateral cracking risk evaluation test]

A lateral cracking risk evaluation test was performed as follows.

<2-1. Production of sample>

<Example B1>

The sample of Example A1 made in Test 1 was used for Example B1.

<Example B2>

In Example B2, a tubular parison having a three-layer structure in which an inner layer (inner diameter: 0.86 mm) included a nylon elastomer (Product name: Grilflex ELG6260, manufactured by Ems-chemie Holding AG), an intermediate layer (inner diameter: 1.07 mm, outer diameter: 1.55 mm) included a nylon (Product name: Grilamid L25, manufactured by Ems-chemie Holding AG), and an outer layer (outer diameter: 1.77 mm) included a nylon elastomer (Product name: Grilflex ELG6260, manufactured by Ems-chemie Holding AG) was produced by extrusion molding, and this tubular parison was disposed in a mold and blow-molded (mold temperature: 115°C, heating time: 30 seconds, pressure: 3.2 MPa) to obtain samples (the number of samples: 5) having an outer diameter at the time of expansion at NP of 6.0 mm.

<Comparative Example B1>

In Comparative Example B1, a tubular parison having a three-layer structure in which an inner layer (inner diameter: 0.86 mm) included a nylon elastomer (Product name: Grilflex ELG6260, manufactured by Ems-chemie Holding AG), an intermediate layer (inner diameter: 1.07 mm, outer diameter: 1.55 mm) included a nylon (Product name: Grilamid L25, manufactured by Ems-chemie Holding AG), and an outer layer (outer diameter: 1.72 mm) included a nylon elastomer (Product name: Grilflex ELG6260, manufactured by Ems-chemie Holding AG) was produced by extrusion molding, and this tubular parison was disposed in a mold and blow-molded (mold temperature: 115°C, heating time: 30 seconds, pressure: 3.0 MPa) to obtain samples (the number of samples: 5) having an outer diameter at the time of expansion at NP of 6.0 mm.

<Comparative Example B2>

In Comparative Example B2, a tubular parison having a three-layer structure in which an inner layer (inner diameter: 0.86 mm) included a nylon elastomer (Product name: Grilflex ELG6260, manufactured by Ems-chemie Holding AG), an intermediate layer (inner diameter: 1.07 mm, outer diameter: 1.55 mm) included a nylon (Product name: Grilamid L25, manufactured by Ems-chemie Holding AG), and an outer layer (outer diameter: 1.67 mm) included a nylon elastomer (Product name: Grilflex ELG6260, manufactured by Ems-chemie Holding AG) was produced by extrusion molding, and this tubular parison was disposed in a mold and blow-molded (mold temperature: 115°C, heating time: 30 seconds, pressure: 3.2 MPa) to obtain samples (the number of samples: 5) having an outer diameter at the time of expansion at NP of 6.0 mm.

<Comparative Example B3>

In Comparative Example B3, a tubular parison having a two-layer structure in which an inner layer (inner diameter: 0.79 mm, outer diameter: 1.10 mm) included a nylon (Product name: Grilamid L25, manufactured by Ems-chemie Holding AG), and an outer layer (outer diameter: 1.62 mm) included a nylon elastomer (Product name: Grilflex ELG6260, manufactured by Ems-chemie Holding AG) was produced by extrusion molding, and this tubular parison was disposed in a mold and blow-molded (mold temperature: 115°C, heating time: 30 seconds, pressure: 3.0 MPa) to obtain samples (the number of samples: 6) having an outer diameter at the time of expansion at NP of 6.0 mm.

<2-2. Measurement of parameter>

<Film thickness>

The film thickness was measured in the same manner as in the film thickness measurement performed in Test 1.

<ADDR>

The ADDR (area draw-down ratio) was an average value of values obtained from the ratio of the cross-sectional area of the tubular parison of each sample to the cross-sectional area of the balloon after blow molding.

<Pressure resistance strength>

The pressure resistance strength was an average value of pressure values obtained when each sample was burst while increasing pressure applied to each sample in increments of 1 atm in a water bath at 37°C.

<Outer diameter at NP>

The outer diameter at NP was an average value of measured values obtained by measuring the outer diameter when each sample was pressurized at 12 atm, which is the recommended expansion pressure (NP), with an outer diameter measuring device (Product name: GT2-H12KLSO (50248), manufactured by KEYENCE CORPORATION) at the time of measuring the pressure resistance strength described above.

<Wall Strength>

The wall strength was a value obtained by dividing the pressure resistance strength of each sample by the film thickness.

<Lateral cracking risk value>

The lateral cracking risk value was acquired as follows.

<Procedure 1>

Using Formula (1) described above, the average balloon film intrinsic refractive index was calculated from the cross-sectional area percentage of each layer of the tubular parison having a three-layer structure.

<Procedure 2>

Each sample after blow molding was cut and spread out to form a rectangular test piece, and the test piece was attached onto a slide glass with a tape adhesive to prepare a measurement sample. A phase difference distribution image of the measurement sample was acquired using a two-dimensional birefringence evaluation system (WPA-200: manufactured by Photonics Lattice, Inc.), and two points in each of three areas, that is, a balloon distal portion, a balloon central portion, and a balloon proximal portion in the test piece of the measurement sample were analyzed to extract a phase difference average value (retardation) of each area.

<Procedure 3>

Birefringence $\Delta$n was calculated from the ratio (retardation average value/film thickness) of the average value of the retardation (any of the balloon distal portion, the balloon central portion, and the balloon proximal portion) obtained in Procedure 2 to the film thickness of the balloon 140 after blow molding measured by a thickness measuring instrument (ID-F125 thickness gauge: manufactured by Mitutoyo Corporation).

<Procedure 4>

A circumferential refractive index nr and an axial refractive index nl were calculated from the birefringence $\Delta n$ obtained in Procedure 3 using the following Formulas (2) and (3). In Formulas (2) and (3), an assumed value (1.501) empirically determined for nylon-based material was substituted into "nd".

<Procedure 5>

Using Formulas (4) to (6) described above, the orientation ratio r% in the circumferential direction and the orientation ratio l% in the axial direction were calculated from the refractive indices in the respective directions obtained in Procedure 4. In Formulas (4) to (6), since the intrinsic birefringence of nylon 6 is 0.072 and the intrinsic birefringence of polyethylene is 0.66, 0.07 was substituted as an approximate value into "n*".

<Procedure 6>

The ratio (r%/l%) of the orientation ratio r% in the circumferential direction to the orientation ratio l% in the axial direction obtained in Procedure 5 was determined, and the obtained value was defined as a lateral cracking risk value. In addition, the above 3) to 6) were calculated for each of the balloon distal portion, the balloon central portion, and the balloon proximal portion to obtain an average value of the lateral cracking risk values of the test piece of each sample, and the average value was acquired as the lateral cracking risk value of each sample.

The specifications of each sample are indicated in Table 2 below. As evaluation indices in the table, from the viewpoints of pressure resistance and operability, a product having a lateral cracking risk value of 1.40 or less, an area draw-down ratio (ADDR) of more than 2.80 and less than 3.00, and a film thickness of 26.6 $\mu$m or more and 36.0 $\mu$m or less was rated as a good product ($\circ$), and a product falling outside these ranges was rated as a defective product ($\times$). The good product is a balloon having excellent pressure resistance and operability by being thinned while suppressing a decrease in pressure resistance strength accompanying an increase in diameter of the balloon.

[Table 2]

| | Example B1 | Example B2 | Comparative example B1 | Comparative example B2 | Comparative example B3 |
|---|---|---|---|---|---|
| Outer diameter (mm) | 6.0 | 6.0 | 6.0 | 6.0 | 6.0 |
| Inner layer inner diameter (mm) | 0.82 | 0.86 | 0.86 | 0.86 | 0.79 |
| Intermediate layer inner diameter (mm) | 1.09 | 1.07 | 1.07 | 1.07 | 1.10 *1 |
| Intermediate layer outer diameter (mm) | 1.56 | 1.55 | 1.55 | 1.55 | |
| Outer layer outer diameter (mm) | 1.73 | 1.77 | 1.72 | 1.67 | 1.62 |
| Number of samples | 10 | 5 | 5 | 5 | 6 |
| ADDR | 2.90 | 2.82 | 2.73 | 2.65 | 2.75 |
| Pressure resistance strength (atm) | 26.3 | 26.8 | 28.8 | 28.2 | 26.3 |
| Outer diameter at time of expansion at NP (mm) | 6.037 | 6.070 | 6.056 | 6.048 | 6.060 |
| Wall strength (atm/mm) | 4782 | 4570 | 5115 | 5264 | 5226 |
| Lateral cracking risk value | 1.36 | 1.45 | 1.46 | 1.52 | 1.56 |
| Evaluation | ○ | × | × | × | - |
| *1 Comparative example 3 indicates outer diameter of inner layer | | | | | |

<2-3. Results>

As indicated in Table 2, in Example B1, the lateral cracking risk value was 1.36, which was 1.40 or less. On the other hand, in each of Example B2 and Comparative Examples B1 to B3, the lateral cracking risk value exceeded 1.40. From this, it was confirmed that Example B1 was a balloon having a reduced risk of lateral cracking as compared with Example B2, Comparative Example 1, and Comparative Example 2.

In Example B1, the film thickness was 33.0 μm, the area draw-down ratio was 2.90, and the lateral cracking risk value was 1.36. In spite of the fact that the outer diameter at the time of expansion at NP was 6.00 mm, it was confirmed that the product had the lowest risk of lateral cracking, had sufficient pressure resistance strength, could secure flexibility by thinning, and had excellent pressure resistance and operability.

In addition, in Example B1, the cross-sectional area percentages of the inner layer, the intermediate layer, and the outer layer were 22.5%, 53.5%, and 24.0%, respectively. It is found that the outer layer amount is decreased and the inner layer amount is increased as compared with a tubular parison (the cross-sectional area percentages of the inner layer, the intermediate layer, and the outer layer are 12.1%, 54.7%, and 33.2%, respectively) of a balloon having an outer diameter at the time of expansion at NP of 4.0 mm, as a target for comparison. It is presumed that, in Example B1, an effect of delaying the expansion timing was exhibited by fixing the intermediate layer amount, increasing the inner layer amount, and decreasing the outer layer amount, and expansion in the axial direction was promoted to reduce the risk of lateral cracking.

In Comparative Example B1 and Comparative Example B2, the inner diameter of the inner layer and the inner and outer diameters of the intermediate layer were fixed (the thicknesses of the inner layer and the intermediate layer were fixed) with reference to Example B2, and only the thickness of the outer layer was reduced to reduce the amount of the outer layer. In Comparative Example B1 and Comparative Example B2, the change in pressure resistance strength was smaller than that in Example B2, and the film thickness decreased in proportion to the decrease in the amount of the outer layer, and the wall strength increased. On the other hand, the area draw-down ratio was less than 2.80, and the lateral cracking risk value increased as the amount of the outer layer decreased. From this, it was confirmed that even if the amount of the outer layer is simply reduced to reduce the thickness, the risk of lateral cracking increases, and thus the cross-sectional area percentages of the inner layer, the intermediate layer, and the outer layer are one of important parameters for thickness reduction and suppressing the risk of lateral cracking.

Comparative Example B3 had a two-layer structure, and thus had the highest lateral cracking risk value. From this, it was confirmed that by forming the balloon into the three-layer structure, the balloon can be fully extended in the axial direction before being inflated in the circumferential direction by enhancing the axial orientation, and thus the risk of lateral cracking can be suppressed as compared with the two-layer structure.

This application is based on Japanese Patent Application No. 2022-155931, filed on September 29, 2022, the entire contents of which are incorporated herein by reference.

Reference Signs List

| | |
|---|---|
| 100 | Balloon catheter |
| 110 | Shaft |
| 120 | Inner tube |
| 130 | Outer tube |
| 140 | Balloon |
| 141 | Inner layer |
| 142 | Intermediate layer |
| 143 | Outer layer |
| 150 | Hub |
| 200 | Mold |
| 300 | Tubular parison |

**Claims**

**1.** A balloon catheter comprising:

a balloon having a main body with an expandable and contractible multilayer film structure, wherein
the balloon includes at least an inner layer and an outer layer,
has an area draw-down ratio (ADDR) of more than 2.80 and less than 3.00, the area draw-down ratio (ADDR) being determined by a ratio of a cross-sectional area of a tubular parison disposed in a mold and blow-molded in a balloon shape to a cross-sectional area of the balloon after blow molding, and
includes a layer provided outside the inner layer and having an elongation rate at a breaking point lower than an elongation rate at a breaking point of the inner layer.

2. The balloon catheter according to claim 1, wherein the area draw-down ratio is 2.91 or more and 2.97 or less.

3. The balloon catheter according to claim 1, wherein a film thickness of the main body of the balloon after blow molding is 26.6 $\mu$m or more and 36.0 $\mu$m or less.

4. The balloon catheter according to claim 1, wherein a film thickness of the main body of the balloon after blow molding is 32.3 $\mu$m or more and 33.0 $\mu$m or less.

5. The balloon catheter according to claim 1, wherein the balloon has a lateral cracking risk value of 1.40 or less, the lateral cracking risk value being obtained from a ratio of a circumferential orientation ratio to an axial orientation ratio.

6. The balloon catheter according to any one of claims 1 to 5, wherein the main body of the balloon has a three-layer structure in which the inner layer, an intermediate layer, and the outer layer are stacked in this order.

7. The balloon catheter according to claim 6, wherein an elongation rate at a breaking point of the intermediate layer is lower than each of the elongation rate at the breaking point of the inner layer and an elongation rate at a breaking point of the outer layer.

8. The balloon catheter according to claim 6, wherein an elongation rate at a breaking point of the outer layer is equal to or more than the elongation rate at the breaking point of the inner layer.

9. The balloon catheter according to claim 8, wherein the elongation rate at the breaking point of the inner layer is equal to the elongation rate at the breaking point of the outer layer.

10. The balloon catheter according to claim 6, wherein in the balloon, cross-sectional area percentages of the inner layer, the intermediate layer, and the outer layer in the tubular parison are 22.5%, 53.5%, and 24.0%, respectively.

11. The balloon catheter according to claim 6, wherein in the main body of the balloon, the inner layer includes a nylon elastomer, the intermediate layer includes a nylon, and the outer layer includes a nylon elastomer.

FIG. 1

EP 4 570 296 A1

# FIG. 2

# FIG. 3

# FIG. 4

EP 4 570 296 A1

# FIG. 5

## INTERNATIONAL SEARCH REPORT

| International application No. |
|---|
| **PCT/JP2023/029666** |

**A. CLASSIFICATION OF SUBJECT MATTER**

**A61M 25/10**(2013.01)i
FI: A61M25/10 500

According to International Patent Classification (IPC) or to both national classification and IPC

**B. FIELDS SEARCHED**

Minimum documentation searched (classification system followed by classification symbols)

A61M25/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2013/047449 A1 (TERUMO KABUSHIKI KAISHA) 04 April 2013 (2013-04-04) paragraphs [0118]-[0120] | 1-11 |
| A | US 2012/0065718 A1 (SIMPSON, John A.) 15 March 2012 (2012-03-15) entire text, all drawings | 1-11 |
| A | JP 2005-518879 A (BOSTON SCIENTIFIC LTD.) 30 June 2005 (2005-06-30) entire text | 1-11 |
| A | JP 2007-528750 A (BOSTON SCIENTIFIC LTD.) 18 October 2007 (2007-10-18) entire text, all drawings | 1-11 |

☐ Further documents are listed in the continuation of Box C.    ☑ See patent family annex.

| | |
|---|---|
| *　Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **05 October 2023** | **24 October 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT
### Information on patent family members

International application No.

**PCT/JP2023/029666**

| Patent document cited in search report | | | Publication date (day/month/year) | Patent family member(s) | | | Publication date (day/month/year) |
|---|---|---|---|---|---|---|---|
| WO | 2013/047449 | A1 | 04 April 2013 | US | 2014/0207171 | A1 | |
| | | | | paragraphs[0226]-[0233] | | | |
| | | | | EP | 2762191 | A1 | |
| | | | | CN | 103764181 | A | |
| | | | | KR | 10-2014-0067002 | A | |
| US | 2012/0065718 | A1 | 15 March 2012 | (Family: none) | | | |
| JP | 2005-518879 | A | 30 June 2005 | US | 2003/0167067 | A1 | |
| | | | | entire text | | | |
| | | | | WO | 2003/074115 | A1 | |
| JP | 2007-528750 | A | 18 October 2007 | US | 2005/0008806 | A1 | |
| | | | | entire text, all drawings | | | |
| | | | | WO | 2005/007230 | A2 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019234784 A

- JP 2022155931 A